# EUROPEAN PATENT SPECIFICATION

(11) **EP 2 557 943 B1**
(45) Date of publication and mention of the grant of the patent: **30.05.2018**
(21) Application number: 11715599.4
(22) Date of filing: 11.04.2011
(51) Int. Cl.: A61K 9/107, A61K 9/00, A61K 31/343, A61K 31/496

(54) **ORAL VETERINARY PHARMACEUTICAL AND NUTRACEUTICAL COMPOSITIONS**
ORALE PHARMAZEUTISCHE UND NUTRAZEUTISCHE ZUSAMMENSETZUNGEN FÜR DIE VETERINÄRMEDIZIN
COMPOSITIONS PHARMACEUTIQUES ET NUTRACEUTIQUES VÉTÉRINAIRES À ADMINISTRATION PAR VOIE ORALE

(30) Priority: 14.04.2010 GB 201006178
(43) Date of publication of application: 20.02.2013
(60) Divisional application: 16194441.8
(73) Proprietor: Vitux Group AS, 0667 Oslo (NO)
(72) Inventor: DRAGET, Kurt Ingar, 0667 Oslo (NO); HAUG, Ingvild Johanne, 0667 Oslo (NO); ENGELSEN, Steinar Johan, 0667 Oslo (NO); SETERNES, Tore, 0667 Oslo (NO)
(74) Representative: Golding, Louise Ann
(86) International application number: PCT/GB2011/000557
(87) International publication number: WO 2011/128627

(56) References cited:
- WO-A1-2007/085840
- WO-A2-2010/041015
- WO-A2-2010/041017
- GB-A- 1 474 629
- US-A- 4 764 383
- US-A1- 2006 165 795
- US-A1- 2007 026 075
- James K. Roush: "Multicenter veterinary practice assessment of the effects of omega-3 fatty acids on osteoarthritis in dogs", J Am Vet Med Assoc., 1 January 2010 (2010-01-01), pages 59-66, XP55021140, Retrieved from the Internet: URL:http://www.hillscampus.it/files/pubbli cazioni/pubbscient/V-javma_236_2 vet assess.pdf [retrieved on 2012-03-07]
- RAATZ S K ET AL: "Enhanced Absorption of n-3 Fatty Acids from Emulsified Compared with Encapsulated Fish Oil", JOURNAL OF THE AMERICAN DIETETIC ASSOCIATION, THE ASSOCIATION, CHICAGO, IL, US, vol. 109, no. 6, 1 June 2009 (2009-06-01), pages 1076-1081, XP026132573, ISSN: 0002-8223, DOI: 10.1016/J.JADA.2009.03.006 [retrieved on 2009-05-21]

## Description

This invention relates to pharmaceutical compositions for oral administration to non-human animals, especially mammals, more particularly domestic pets, especially carnivorous pets, e.g. dogs and cats.

Oral veterinary drugs are routinely presented in the same forms as oral drugs for humans, i.e. as tablets. Many animals however are highly adept at avoiding consuming tablets, even where camouflaged within their feed.

US 2007/0026075 discloses gelled donepezil compositions which are suitable for animals and which avoid unpleasant taste and "tongue-biting" sensations.

Domestic animals moreover frequently suffer from diseases associated with lipid and vitamin malnutrition. This can be caused by disease of the small intestine or exocrine pancreatic insufficiency (EPI) and may be treated by providing a diet enriched with highly digestible fats, nutritional supplementation with pancreatic extracts and vitamin supplementation. EPI, which is more common with dogs than cats, is frequently caused by pancreatic acinar atrophy in dogs and by pancreatitis in cats. Impaired fat uptake is also associated with impairment of uptake of fat-soluble vitamins.

We have now found that oral administration of drugs (pharmaceuticals) or nutritional supplements (nutraceuticals), such as vitamins, minerals, lipids, and digestive enzymes, may be facilitated by presenting the pharmaceutical or nutraceutical composition in the form of a physiologically tolerable, gelled, oil-in-water emulsion. The uptake of lipid nutrients from such compositions has moreover surprisingly been found to be higher with such gelled emulsions than with lipid-filled capsules. Due to their more food-like texture, and enhanced with taste or odour attractants, consumption avoidance may be reduced or eliminated.

Thus viewed from one aspect the invention provides an oral veterinary pharmaceutical composition comprising a physiologically tolerable gelled oil-in-water emulsion further comprising an attractant which is a chicken, beef, pork, lamb, rabbit or fish flavouring, and a veterinary drug, wherein the veterinary drug is dispersed in the water phase of the emulsion.

Viewed from a further aspect the invention provides an oral veterinary pharmaceutical composition comprising a physiologically tolerable gelled oil-in-water emulsion further comprising an attractant which is a chicken, beef, pork, lamb, rabbit or fish flavouring, and a veterinary drug, wherein the veterinary drug is dispersed in the water phase of the emulsion, for use in treatment of a non-human mammalian subject to combat a condition responsive to said veterinary drug.

Viewed from a still further aspect the invention provides an oral veterinary pharmaceutical composition comprising a physiologically tolerable gelled oil-in-water emulsion further comprising an attractant which is a chicken, beef, pork, lamb, rabbit or fish flavouring, and a veterinary drug, wherein the veterinary drug is dispersed in the water phase of the emulsion, for use in a method of treatment of a non-human mammalian subject by oral administration to said subject of an effective amount of a veterinary drug.

Also described herein is a method of dietary supplementation of a non-human mammalian animal subject which comprises orally administering to said subject at least one dietary supplement in a physiologically tolerable gelled oil-in-water emulsion.

The compositions of and used in the methods of the invention contain an attractant, a material the taste or odour of which is attractive to the animal recipient. As the attractant, a chicken, beef, pork, lamb, rabbit or fish flavouring is used. Typically this will be a proteinaceous and/or lipid material, or a protein hydrolysate or oligopeptide. Where the animal recipient is feline, the attractant may conveniently be a fish oil, e.g. salmon oil.

Examples of nutrients that may be included in the compositions of the invention include lipids, (especially triglycerides and phospholipids, typically of plant or marine animal origin), vitamins, minerals, folic acid, and digestive enzymes, especially pancreatic digestive enzymes, e.g. lipases, proteases, amylases or a combination thereof.

The veterinary drug substance may be any veterinary drug substance that is suitable for oral administration. The veterinary drug is dispersed in the water phase of the emulsion and optionally may be presented in both the water and oil phases of the gelled emulsion, e.g. in dissolved or dispersed form. The drug substance will typically be present in the gelled emulsion at 10 to 100% wt of the conventional oral daily dosage, especially 50 or 100% wt.

Examples of appropriate drug substances include antibiotics, antifungals, analgesics, antihelminthics, antidiabetics, oral contraceptives, hormones, antiinflammatory drugs, cardiovascular drugs, antihistamines, antidepressants and minerals, vitamins and essential fatty acids in triglyceride, monoacylglycerol, ethyl ester, phospholipid or free fatty acid form.

Particular examples of appropriate drug substances include: antibiotics e.g.penicillin V, cloxacillin, dicloxacillin, amoxicillin, ampicillin, cephalexin, cefaclor, cefdinir, doxycycline, doxycycline, enrofloxacin, oxytetracycline, azithromycin, erythromycin, clindamycin palmitate, trimethoprim, sulfamathoxazole (SMO), gentamicin, metronidazole, amoxycillin/clavulanic acid; antifungals e.g. polyene; antifungals e.g. amphotericin B, nystatin, pimaricin, imidazoles e.g. clotrimazole, miconazole, econazole, ketaconazole, itraconazole, fluoconazole, flucytocine and griseofulvin; analgesics e.g. morphine, butorphanol, hydromorphone, oxycodone; antihelminthics; clamoxyquine, dichlorophene, ivermectin, milbemycin oxime, ponazuril, mebendazole, flubendazole, fenbendazole, febantel, pyrantel tartrate, morantel, piperazine; antidiabetics e.g. glipizide; contraceptives e.g. megestrol acetate, mibolerone, proligestone, medroxyprogesterone acetate; hormones e.g. glucocorticoids, thyroid hormones, progesterones, sex hormones, melatonin; anti inflammatory drugs e.g. firocoxib, meloxicam, carprofen, ketoprofen, etodolac, aspirin; cardiovascular drugs e.g. digoxin,digitoxin, amrinone, milrinone, primobendan, enalapril, lisinopril, benazepril, hydralazine, amlodipine; antihistamines e.g. diphenhydramine, hydroxyzine, clorpheniramine, cyproheptadine, terfenadine, clemastine, trimeprazine; antidepressants like SSRI's; fluoxetine, paroxetine, certraline, citalopram; minerals and vitamins, e.g. zinc, calcium, retinyl palmitate, fat soluble vitamins (A, D, E, K) and water soluble vitamins (B-complex, C, folate etc) and essential fatty acids e.g. omega-3 fatty acids.

The gelled emulsion compositions of the invention will preferably be in dose unit form, with each dose unit having a weight of 50 to 5000 mg, especially 100 to 3000 mg, particularly 400 to 2000 mg, more particularly 600 to 1500 mg.

Although the compositions may be coated, e.g. as described in WO2007/085835, in order that the attractant should immediately be perceptible to the animal recipient, the dose units of the composition of the invention will preferably be uncoated, i.e. not within a capsule or shell-coating. Accordingly, to avoid water loss during storage, the dose units will conveniently be individually packaged, e.g. in foil wrappers or in the blisters of a blister pack.

Where a coating is used, this is preferably a gelatin coating. This can be applied before or after the emulsion has set.

Besides the components already mentioned, the compositions of the invention may optionally contain pH modifiers, sugars or other carbohydrates, viscosity modifiers, and colorants.

The oil phase of the oil-in-water emulsion may be any physiologically tolerable lipid, e.g. free fatty acids (or salts thereof), fatty acid esters such as mono-, di- and triacylglycerides and phospholipids, for example plant or animal oils, especially plant and marine animal oils. Particularly preferably an oil is used which is high in omega-3, omega-6 or omega-9 essential fatty acids, especially omega-3 essential fatty acids, more especially EPA and DHA. In this way the oil phase itself is a highly bioavailable source of nutrient lipids. It is especially preferred that the oil phase include free essential fatty acids (or physiologically tolerable salts thereof) and/or monoacylglycerides of essential fatty acids to facilitate essential fatty acid uptake from the gut.

Examples of omega-3 acids include α-linolenic acid (ALA), stearidonic acid (SDA), eicosatrienoic acid (ETE), eicosatetraenoic acid (ETA), eicosapentaenoic acid (EPA), docosapentaenoic acid (DPA), docosahexaenoic acid (DHA), tetracosapentaenoic acid and tetracosahexaenoic acid. Examples of omega-6 acids include linoleic acid, gamma-linolenic acid, eicosadienoic acid, dihomo-gamma-linolenic acid (DGLA), arachidonic acid (AA), docosadienoic acid, adrenic acid, docosapentaenoic acid, and calendic acid. Examples of omega-9 acids include oleic acid, eicosenoic acid, mead acid, erucic acid and nervonic acid.

The essential fatty acids may form part or the whole of the oil phase in the gelled emulsion, preferably at least 10% wt, more especially at least 50% wt, particularly at least 80% wt. of that phase. They may be used as single compounds or as compound mixtures, e.g. plant or marine oils.

Where a free fatty acid is used, this is preferably wholly or partially in salt form, and preferably constitutes 5 to 75% wt, especially 10 to 35% wt. of the essential fatty acid in the oil phase. Quite surprisingly, the soapy taste of the free fatty acid is masked by presentation in the gelled oil-in-water emulsion form. Where free fatty acids or salts thereof are used, it is particularly preferable to use these in conjunction with monoacylglycerides or diacylglycerides such that the molar ratio of free fatty acid to monoacylglyceride is about 2:1, e.g. 1.9:1 to 2.1:1, or free fatty acid to diacylglyceride is about 1:1, e.g. 0.9:1 to 1.1:1.

The oil phase of the oil-in-water emulsion may also contain solubilisers in order to increase the solubility of the drug substance in the oil phase. Suitable solubilisers would be known to a person skilled in the art and include Chremophor EL™, castor oil, Tween 80™, Solutol™ HS15, Lutrol™ and Olestra.

The aqueous phase of the gelled emulsion will contain water and a physiologically tolerable gelling agent, e.g. a hydrocolloid such as gelatin, alginate, carrageenan or a pectin. Such gelling agents and their gel-forming properties are well known. See for example Phillips GO and Williams PA (Eds.) Handbook of hydrocolloids, Woodhead Publishing, Cambridge (2000). When the compositions of the invention comprise enzymes such as one or more of lipases, proteases and amylases, the gelling agent is preferably pectin. The use of gelatin is especially preferred for use in the invention. Besides water and the gelling agent, the aqueous phase of the gelled emulsion may contain other water-soluble components, e.g. vitamins, minerals, pH modifiers, viscosity modifiers, antioxidants, colorants, flavours, water-soluble drug substances, etc. as desired.

Particularly preferably the gelled emulsions contain vitamins and optionally also minerals. Examples of such components include calcium, folic acid, iron, vitamin B12 and other B vitamins complexes, and vitamins A, D, E, K, and retinyl palmitate. Desirably these should be included at 10 to 100% of the recommended daily dose. If the composition contains fish oils, iron is less preferred due to its potentially oxidative effects.

The weight ratio of the lipid phase to the aqueous phase in the gelled emulsions is preferably 1:19 to 3:1, especially 35:65 to 1:1, particularly 2:3 to 1:1.

Emulsion formation may be effected by conventional techniques; however emulsification under a non-oxidising gas, e.g. nitrogen, is preferred. Likewise, the components of the emulsion are preferably degassed before emulsification and handling and packaging of the set emulsion is preferably performed under such a gas.

The gelled emulsions of and used according to the invention may be produced as described in WO 2007/085835 and WO 2007/085840 and PCT/GB2009/002404 and PCT/GB2009/002406.

The gelled emulsions may if desired be more than biphasic. Thus a water-in-oil emulsion may be emulsified with an aqueous gelling agent phase to produce a water-in-oil-in-water double emulsion, or two oil-in-water emulsions with different oil phases may be combined and intimately mixed before gelling onset.

The invention will now be illustrated further with reference to the following nonlimiting Examples.

### Example 1

### Basic recipe for veterinary dosage form:

**Components (% by wt.)**

| | |
|---|---|
| Gelatin | 7.5 % |
| Gum arabicum | 3.5 % |
| Fish protein hydrolysate | 0 - 22 % |
| Chicken broth powder | 0 - 22 % |
| Salt | 0 - 2 % |
| Flavouring | 1 % |
| Colour | 0.5 % |
| Preservatives | 0 - 3 % |
| Oil | 5 to 35% |
| Water to | 100 % |

Oil (e.g. plant or marine animal oil) is emulsified with the aqueous phase containing gelatin, gum arabicum, proteins, salt and preservatives and the emulsion is poured in aliquots of 1.5 g into elongate moulds lined with a metal/plastics laminate blister tray and allowed to set. The blister tray is thermally sealed with a metal/plastics foil cover sheet.

### Example 2

### Canine and Feline Nutraceutical with essential fatty acids

**Components (% by wt.)**

| | |
|---|---|
| Gelatin | 7.5 % |
| Gum arabicum | 3.5 % |
| Fish protein hydrolysate | 0 - 22 % |
| Chicken broth powder | 0 - 22 % |
| Salt | 0 - 2 % |
| Flavouring | 1 % |
| Colour | 0.5 % |
| Preservatives | 0 - 3 % |
| Triglycerides* | 0 - 40 % |
| Ethyl esters of fatty acids* | 0 - 40 % |
| Free fatty acids* | 0 - 40 % |
| Monoacylglycerides* | 0 - 40 % |
| Phospholipids* | 0 - 40 % |
| Water to | 100 % |

| | |
|---|---|
| * Total not to exceed 40% | |

Free fatty acids (e.g. omega-3 acids, in particular a mixture of DHA and EPA), ethyl esters, triacylglycerides, phospholipids, and/or monoacylglycerides of poly and mono unsaturated fatty acids are emulsified into the aqueous phase and the emulsion is poured in aliquots of 1.5 g into elongate moulds lined with a metal/plastics laminate blister tray and allowed to set. The blister tray is thermally sealed with a metal/plastics foil cover sheet.

### Example 3

### Canine and Feline Nutraceutical with essential fatty acids and vitamins

**Components (% by wt.)**

| | |
|---|---|
| Gelatin | 7.5 % |
| Gum arabicum | 3.5 % |
| Fish protein hydrolysate | 0 - 22 % |
| Chicken broth powder | 0 - 22 % |
| Salt | 0 - 2 % |
| Flavouring | 1 % |
| Colour | 0.5 % |
| Preservatives | 0 - 3 % |
| Triglycerides* | 0 - 40 % |
| Ethyl esters of fatty acids* | 0 - 40 % |
| Free fatty acids* | 0 - 40 % |
| Monoacylglycerides* | 0 - 40 % |
| Phospholipids* | 0 - 40 % |
| Vitamins | 0.01 - 1 % |
| Water to | 100 % |

| | |
|---|---|
| * Total not to exceed 40% | |

Free fatty acids (e.g. omega-3 acids, in particular a mixture of DHA and EPA), ethyl esters, triacylglycerides, phospholipids, and/or monoacylglycerides of poly and mono unsaturated fatty acids are emulsified together with the lipid-soluble vitamins into the aqueous phase and the emulsion is poured in aliquots of 1.5 g into elongate moulds lined with a metal/plastics laminate blister tray and allowed to set. The blister tray is thermally sealed with a metal/plastics foil cover sheet.

### Example 4

### Canine and Feline antibiotics

**Components (% by wt.)**

| | |
|---|---|
| Gelatin | 7.5 % |
| Gum arabicum | 3.5 % |
| Fish protein hydrolysate | 0 - 22 % |
| Chicken broth powder | 0 - 22 % |
| Salt | 0 - 2 % |
| Flavouring | 1 % |
| Colour | 0.5 % |
| Preservatives | 0 - 3 % |
| Oil | 5 to 35% |
| Ciprofloxacin | 180 mg/tablet* |
| Water to | 100 % |

| | |
|---|---|
| * Suitable for a 15kg animal - to be adjusted for smaller/heavier animals. | |

Oil (e.g. plant or marine animal oils), together with ciprofloxacin, is emulsified into the aqueous phase and the emulsion is poured in aliquots of 1.5 g into elongate moulds lined with a metal/plastics laminate blister tray and allowed to set. The blister tray is thermally sealed with a metal/plastics foil cover sheet.

### Example 5

### Canine and Feline antifungals

**Components (% by wt.)**

| | |
|---|---|
| Gelatin | 7.5 % |
| Gum arabicum | 3.5 % |
| Fish protein hydrolysate | 0 - 22 % |
| Chicken broth powder | 0 - 22 % |
| Salt | 0 - 2 % |
| Flavouring | 1 % |
| Colour | 0.5 % |
| Preservatives | 0 - 3 % |
| Oil | 5 to 35% |
| Griseofulvin | 375 mg* |
| Water to | 100 % |

| | |
|---|---|
| * Suitable for a 15kg animal - to be adjusted for smaller/heavier animals. | |

Oil (e.g. plant or marine animal oils), together with griseofulvin is emulsified into the aqueous phase and the emulsion is poured in aliquots of 1.5 g into elongate moulds lined with a metal/plastics laminate blister tray and allowed to set. The blister tray is thermally sealed with a metal/plastics foil cover sheet.

### Example 6

### Canine and Feline Nutraceutical with digestive enzyme and nutrients

**Components (% by wt.)**

| | |
|---|---|
| Gelatin | 7.5 % |
| Gum arabicum | 3.5 % |
| Fish protein hydrolysate | 0 - 22 % |
| Chicken broth powder | 0 - 22 % |
| Salt | 0 - 2 % |
| Flavouring | 1 % |
| Colour | 0.5 % |
| Preservatives | 0 - 3 % |
| Triglycerides* | 0 - 40 % |
| Ethyl esters of fatty acids* | 0 - 40 % |
| Free fatty acids* | 0 - 40 % |
| Monoacylglycerides* | 0 - 40 % |
| Phospholipids* | 0 - 40 % |
| Vitamins | 0.01 - 1 % |
| Pancreatic Lipase | 10 000 U |
| Water to | 100 % |

| | |
|---|---|
| * Total not to exceed 40% | |

Free fatty acids (e.g. omega-3 acids, in particular a mixture of DHA and EPA), ethyl esters, triacylglycerides, phospholipids, and/or monoacylglycerides of poly and mono unsaturated fatty acids are emulsified together with the lipid-soluble vitamins into the aqueous phase with pancreatic lipase and the emulsion is poured in aliquots of 1.5 g into elongate moulds lined with a metal/plastics laminate blister tray and allowed to set. The blister tray is thermally sealed with a metal/plastics foil cover sheet.

## Claims

1. An oral veterinary pharmaceutical composition comprising a physiologically tolerable gelled oil-in-water emulsion further comprising an attractant which is a chicken, beef, pork, lamb, rabbit or fish flavouring, and a veterinary drug, wherein the veterinary drug is dispersed in the water phase of the emulsion.

2. A composition as claimed in claim 1 containing a digestive enzyme.

3. A composition as claimed in claim 1 or claim 2 containing a protein hydrolysate.

4. A composition as claimed in any one of claims 1 to 3 containing a salmon flavouring.

5. A composition as claimed in any one of the preceding claims wherein the oil phase of said emulsion comprises an omega-3 fatty acid or ester or salt thereof.

6. A composition as claimed in any one of the preceding claims wherein the physiologically tolerable gelled oil-in-water emulsion is chewable.

7. An oral veterinary pharmaceutical composition as defined in claim 1 comprising a veterinary drug for use in treatment of a non-human mammalian subject to combat a condition responsive to said veterinary drug.

8. A composition for use as claimed in claim 7 wherein the oil phase of said emulsion comprises an omega-3 fatty acid ester or salt thereof.

9. A composition as claimed in claim 7 or claim 8 for use in a method of treatment of a non-human mammalian subject by oral administration of an effective amount of said veterinary drug.

10. A composition as claimed in claim 9 for use in a method of treatment of a disease of the small intestine or exocrine pancreatic insufficiency.

## Patentansprüche

1. Orale pharmazeutische Zusammensetzung für die Veterinärmedizin, umfassend eine physiologisch tolerierbare gelierte Öl-in-Wasser-Emulsion, weiter umfassend einen Lockstoff, der ein Huhn-, Rinder-, Schweine-, Lamm-, Kaninchen- oder Fisch-Geschmack ist, und ein Veterinärarzneimittel, wobei das Veterinärarzneimittel in der Wasser-Phase der Emulsion dispergiert ist.

2. Zusammensetzung nach Anspruch 1, enthaltend ein Verdauungsenzym.

3. Zusammensetzung nach Anspruch 1 oder Anspruch 2, enthaltend ein Proteinhydrolysat.

4. Zusammensetzung nach einem der Ansprüche 1 bis 3, enthaltend einen Lachs-Geschmack.

5. Zusammensetzung nach einem der vorstehenden Ansprüche, wobei die Öl-Phase der Emulsion eine omega-3-Fettsäure oder Ester oder Salz davon umfasst.

6. Zusammensetzung nach einem der vorstehenden Ansprüche, wobei die physiologisch tolerierbare gelierte Öl-in-Wasser-Emulsion kaubar ist.

7. Orale pharmazeutische Zusammensetzung für die Veterinärmedizin, wie in Anspruch 1 definiert, umfassend ein Veterinärarzneimittel zur Verwendung bei der Behandlung von einem nicht-menschlichen Säugerpatienten zum Bekämpfen eines Zustands, der auf das Veterinärarzneimittel anspricht.

8. Zusammensetzung zur Verwendung nach Anspruch 7, wobei die Öl-Phase der Emulsion einen omega-3-Fettsäureester oder Salz davon umfasst.

9. Zusammensetzung nach Anspruch 7 oder Anspruch 8 zur Verwendung in einem Verfahren zur Behandlung von einem nicht-menschlichen Säugerpatienten durch orale Verabreichung einer wirksamen Menge des Veterinärarzneimittels.

10. Zusammensetzung nach Anspruch 9 zur Verwendung in einem Verfahren zur Behandlung einer Erkrankung des Dünndarms oder exokriner Pankreasinsuffizienz.

## Revendications

1. Composition pharmaceutique vétérinaire orale comprenant une émulsion huile-dans-eau gélifiée physiologiquement tolérable comprenant en outre un agent d'attraction qui est un arôme de poulet, de boeuf, de porc, d'agneau, de lapin ou de poisson, et un médicament vétérinaire, dans laquelle le médicament vétérinaire est dispersé dans la phase aqueuse de l'émulsion.

2. Composition selon la revendication 1 contenant une enzyme digestive.

3. Composition selon la revendication 1 ou la revendication 2 contenant un hydrolysat de protéine.

4. Composition selon l'une quelconque des revendications 1 à 3 contenant un arôme de saumon.

5. Composition selon l'une quelconque des revendications précédentes dans laquelle la phase huileuse de ladite émulsion comprend un acide gras oméga-3 ou un ester ou sel de ceux-ci.

6. Composition selon l'une quelconque des revendications précédentes dans laquelle l'émulsion huile-dans-eau gélifiée physiologiquement tolérable peut être mâchée.

7. Composition pharmaceutique vétérinaire orale selon la revendication 1 comprenant un médicament vétérinaire pour une utilisation dans le traitement d'un sujet mammifère non humain pour lutter contre une affection sensible audit médicament vétérinaire.

8. Composition pour une utilisation selon la revendication 7 dans laquelle la phase huileuse de ladite émulsion comprend un ester d'acide gras oméga-3 ou un sel de celui-ci.

9. Composition selon la revendication 7 ou la revendication 8 pour une utilisation dans un procédé de traitement d'un sujet mammifère non humain par administration orale d'une quantité efficace dudit médicament vétérinaire.

10. Composition selon la revendication 9 pour une utilisation dans un procédé de traitement d'une maladie de l'intestin grêle ou d'une insuffisance pancréatique exocrine.
